# EUROPÄISCHE PATENTANMELDUNG

(11) **EP 3 184 502 A1**
(43) Veröffentlichungstag der Anmeldung: **28.06.2017**
(21) Anmeldenummer: 15202369.3
(22) Anmeldetag: 23.12.2015
(51) Int. Cl.: C07C 29/70, C07C 29/94

(54) **VERFAHREN ZUR HERSTELLUNG VON ALKALIMETALLETHANOLAT**

(71) Anmelder: Evonik Degussa GmbH, 45128 Essen (DE)
(72) Erfinder: REIMANN, Sebastian, 53111 Bonn (DE); NISSEN, Felix, 48301 Nottuin (DE); PERA, Lars, 56598 Hammerstein (DE)

(57) **Zusammenfassung**

Die vorliegende Erfindung betrifft ein Verfahren zur Herstellung von Alkalimetallethanolat. Das Verfahren zeichnet sich dadurch aus, dass das damit erhaltene Alkalimetallethanolat besonders farbstabil ist. Es wird außerdem auch ein Verfahren zur Lagerung von Alkalimetallethanolat beschrieben.

## Beschreibung

Die vorliegende Erfindung betrifft ein Verfahren zur Herstellung von Alkalimetallethanolat. Das Verfahren zeichnet sich dadurch aus, dass das damit erhaltene Alkalimetallethanolat besonders farbstabil ist. Außerdem betrifft die vorliegende Erfindung auch ein Verfahren zur Lagerung von Alkalimetallethanolat.

### Hintergrund der Erfindung

Alkalimetallalkoholate sind industriell vielfach genutzte Chemikalien. Sie werden zum Beispiel als Katalysatoren bei der Synthese vieler organischer Verbindungen verwendet, aber auch bei der Herstellung von Biodiesel aus pflanzlichen Fetten und Ölen.

Gemäß Stand der Technik können Alkalimetallalkoholate durch Umsetzung des entsprechenden Alkohols mit dem Alkalimetall gewonnen werden. Das Alkalimetall kann dabei im elementaren Zustand oder als Amalgam eingesetzt werden, wobei die letztere eine kostengünstige Variante dieses Reaktionsweges darstellt (US 2,069,403; DE 198 02 013 A1). Während die Reaktion des elementaren Alkalimetalls mit Alkoholen gerade im Falle des Methanols und des Ethanols unter stürmischer Wasserstoffentwicklung vonstatten geht, ist die Reaktion des Alkalimetallamalgams mit dem Alkohol langsamer. Deshalb werden in den Fällen, in denen eine schnelle Umsetzung des Alkalimetallamalgams gewünscht ist, oft Katalysatoren zugefügt, um die Reaktion zu beschleunigen und deren Wirtschaftlichkeit zu erhöhen. Im Stand der Technik werden eine Vielzahl an Katalysatoren dafür vorgeschlagen, zum Beispiel Graphit oder Fe/Graphit (US 2,069,403; DE 973 323; US 2,761,880 A1; EP 1 195 369 A1), Carbide, Nitride oder Carbonitride von Übergangsmetallen (DE 198 02 013 A1), Carbide und Nitride von Chrom, Molybdän und Wolfram sowie Titancarbiden (EP 0 810 193 A2, US 5,262,133 A), Schwermetalloxid/ Anthrazit (EP 0 177 768 A1), poröses Eisen (EP 1 533 291 A1).

Obwohl die vorbeschriebenen Verfahren grundsätzlich auch im Falle längerkettiger Alkohole eingesetzt werden können, sind sie besonders bei der Herstellung von Alkoholaten mit nicht mehr als vier Kohlenstoffatomen, ganz besonders bei Methanolaten und Ethanolaten, relevant. Dabei wurde folgendes Problem beobachtet: Im Falle der Herstellung von Alkalimetallethanolaten durch die vorbeschriebenen Prozesse des Standes der Technik ist das erhaltene Alkalimetallethanolat nicht farbstabil und vergilbt bei der Lagerung. Dieser Vergilbungseffekt weist auf Zersetzungsprodukte und/oder Verunreinigungen hin und ist gerade bei kommerziell vertriebenen Chemikalien unerwünscht.

Die Aufgabe der vorliegenden Erfindung bestand deshalb darin, ein Verfahren zur Herstellung von Alkalimetallethanolaten zur Verfügung zu stellen, welche sich im Vergleich zu den mittels der Verfahren des Standes der Technik hergestellten Alkalimetallethanolaten durch eine höhere Farbstabilität auszeichnen.

Es wurde nun überraschend ein Verfahren gefunden, welches die vorstehend definierte Aufgabe löst.

### Detaillierte Beschreibung der Erfindung

Die vorliegende Erfindung betrifft demnach ein Verfahren zur Herstellung von Alkalimetallethanolat mit hoher Farbstabilität, wobei man
a) Alkalimetall mit Ethanol umsetzt, wodurch Alkalimetallethanolat erhalten wird;
b) das in Schritt a) erhaltene Alkalimetallethanolat mit einem Stabilisator **S** vermischt, wobei **S** ausgewählt aus der Gruppe bestehend aus
   Verbindungen **(I-A)** der Struktur NH₂R¹ und Salze **(I-B)** umfassend das Kation [H₃NR¹]^{m+},
      wobei m eine ganze Zahl ≥ 1 ist,
      und wobei R¹ ausgewählt aus der Gruppe bestehend aus -NH-Phenyl, -Phenyl, -(C=O)-NH₂, -OH, verzweigter oder unverzweigter Alkylrest, in welchem mindestens ein Wasserstoffatom durch eine Gruppe ausgewählt aus -NH₂, -OH und im Falle der Salze **(I-B)** auch durch -NH₃⁺ substituiert sein kann, ist,
   Verbindungen **(II-A)** der Struktur NR²R³R⁹ und Salze **(II-B)** umfassend das Kation [HNR²R³R⁹]ⁿ⁺,
      wobei n eine ganze Zahl ≥ 1 ist,
      und wobei R², R³ unabhängig voneinander jeweils ein Alkylrest sind,
      und wobei R⁹ ausgewählt aus der Gruppe bestehend aus -OH, verzweigter oder unverzweigter Alkylrest, in welchem mindestens ein Wasserstoffatom durch eine Gruppe ausgewählt aus -NH₂, -OH und im Falle der Salze **(II-B)** auch durch -NH₃⁺ substituiert sein kann, ist,
   Verbindungen **(III-A)** der Struktur R⁴-COOR⁵, Carboxylate **(III-B)** der Struktur R⁴-COO⁻, sowie Orthoester **(III-C)** der Struktur R⁴-C(OR⁶)₃,
      wobei R⁴ ausgewählt aus der Gruppe bestehend aus Wasserstoff, verzweigter oder unverzweigter Alkyl- oder Alkenylrest, in welchem mindestens ein an ein Kohlenstoffatom gebundenes Wasserstoffatom durch einen Rest ausgewählt aus der Gruppe bestehend aus -SH, -SCH₃, -COOR*, -NH₂ und im Falle der Carboxylate **(III-B)** auch durch -NH₃⁺, -COO⁻ substituiert sein kann, ist,
      und wobei R⁵, R* unabhängig voneinander jeweils Wasserstoff oder ein Alkylrest sind,
      und wobei R⁶ ein Alkylrest ist,
   Salze von Alkalimetallkationen mit dem Anion [HSO₃]⁻ oder mit Anionen der allgemeinen Formeln **(IV-A)** und **(IV-B)** mit 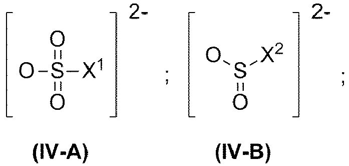 wobei X¹ und X² unabhängig voneinander jeweils ausgewählt sind aus der Gruppe bestehend aus -O, -S, ; 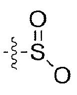 sind, und wobei X¹ auch die Bedeutung 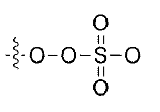 haben kann,
   Alkylthiolen, in denen ein an ein Kohlenstoffatom gebundenes Wasserstoffatom durch eine gegebenenfalls alkylierte und gegebenenfalls protonierte Aminogruppe ersetzt sein kann,
   Formaldehyd,
   Zucker,
      ist.

Unter "Alkalimetallethanolat mit hoher Farbstabilität" ist erfindungsgemäß die nach Schritt b) des erfindungsgemäßen Verfahrens erhaltene Mischung aus dem in Schritt a) erhaltenen Alkalimetallethanolat und dem Stabilisator **S** zu verstehen.

In einem ersten Schritt a) des erfindungsgemäßen Verfahrens wird Alkalimetall mit Ethanol umgesetzt, wodurch Alkalimetallethanolat erhalten wird. Das Alkalimetall ist dabei insbesondere ausgewählt aus Natrium, Kalium, bevorzugt ist das Alkalimetall Natrium.
Die Umsetzung von Alkalimetall mit Ethanol ist nicht besonders beschränkt und nach dem Fachmann bekannten Verfahren möglich, wie zum Beispiel in der DE 973 323, DE 198 02 013 A1 beschrieben.

Insbesondere kann das Alkalimetall in Schritt a) dabei als elementares Alkalimetall oder als Alkalimetallamalgam eingesetzt werden.
Da die Verwendung von elementarem Alkalimetall teuer ist, wird das Alkalimetall, insbesondere im Falle dass es sich dabei um Natrium oder Kalium, noch bevorzugter Natrium handelt, im Schritt a) bevorzugt als Alkalimetallamalgam eingesetzt.

Dieses Alkalimetallamalgam wird beispielsweise bei der Durchführung einer Chloralkalielektrolyse im Rahmen des Amalgamverfahrens erhalten, weshalb seine Verwendung kostengünstiger im Vergleich zum elementaren Alkalimetall ist. Die Durchführung des Amalgamverfahrens und damit auch die Herstellung von Alkalimetallamalgam ist dem Fachmann bekannt und zum Beispiel beschrieben in A. F. Holleman, E. Wiberg, N. Wiberg: Lehrbuch der Anorganischen Chemie. 102. Auflage. de Gruyter, Berlin 2007. Im Falle dass es sich bei dem Alkalimetall um Natrium handelt, wird zum Beispiel aus einer Natriumchloridlösung elektrolytisch an einer Titananode Chlorgas und an einer Amalgamkathode Natrium dargestellt. Das an der Kathode dargestellte Natrium geht dann direkt in das Amalgam über. Das so dargestellte Natriumamalgam kann dann direkt dem Schritt a) des erfindungsgemäßen Verfahrens zugeführt werden.

Im Schritt a) des erfindungsgemäßen Verfahrens wird in dieser bevorzugten Ausführungsform das Alkalimetallamalgam, bevorzugt das Natriumamalgam, dann mit Ethanol umgesetzt. Auch diese Umsetzung ist im Stand der Technik beschrieben (DE 973 323). Erfindungsgemäß wird dieser Schritt bevorzugt in Gegenwart eines heterogenen Katalysators durchgeführt. Als heterogener Katalysator kommen alle im Stand der Technik, zum Beispiel die in der US 2,069,403, DE 973 323; US 2,761,880 A1, EP 1 195 369 A1, DE 198 02 013 A1, EP 0 810 193 A2, US 5,262,133 A, EP 0 177 768 A1, EP 1 533 291 A1 beschriebenen, in Frage. Insbesondere umfasst dieser heterogene Katalysator mindestens Kohlenstoff, zum Beispiel Graphit, und/oder ein Metall ausgewählt aus Aluminium, Übergangsmetall, noch bevorzugter Kohlenstoff, zum Beispiel Graphit, und ein Metall ausgewählt aus Aluminium, Übergangsmetall. Das Übergangsmetall ist dabei bevorzugt ausgewählt aus denen der Nebengruppen Vb (= Vanadium-Gruppe) und Vlb (= Chrom-Gruppe). Besonders bevorzugt ist das Übergangsmetall Molybdän. In einer bevorzugten Ausführungsform handelt es sich um einen Trägerkatalysator, bei welchem eines der vorgenannten Übergangsmetalle, insbesondere ein Metall ausgewählt aus der Vanadium-Gruppe, der Chrom-Gruppe, wobei das Metall bevorzugt Mo ist, auf einem Träger wie insbesondere Kohlenstoff (Graphit zum Beispiel) geträgert ist.

Nach Durchführung des Schrittes a) des erfindungsgemäßen Verfahrens wird dann das Alkalimetallethanolat erhalten. Dieses liegt bevorzugt als ethanolische Lösung vor, und kann insbesondere eine Konzentration von Natriumethanolat in Ethanol von 5 bis 31 Gew.-%, bevorzugt 10 bis 21 Gew.-%, bevorzugter 14 bis 15 Gew-% aufweisen. Die Konzentration kann durch destillative Entfernung oder durch Zugabe von Ethanol variiert werden, zum Beispiel von 14 Gew.-% auf 21 Gew.-%.

In einer bevorzugten Ausführungsform können Quecksilberreste in der Alkalimetallethanolatlösung nach dem Fachmann geläufigen Standardmethoden entfernt werden, zum Beispiel mit dem in EP 2 656 905 A1 beschriebenen Verfahren.

Im Rahmen der vorliegenden Erfindung wurde nun beobachtet, dass das im Schritt a) erhaltene Alkalimetallethanolat eine unerwünschte Tendenz aufweist, sich über die Zeit gelb bis dunkelbraun zu verfärben. Dieses Verhalten ist insbesondere dann nachteilig, wenn es gelagert werden muss, bevor es einem weiteren Einsatz, beispielsweise als Katalysator in einer chemischen Synthesereaktion oder bei der Herstellung von Biodiesel zugeführt wird.

Im Schritt b) des erfindungsgemäßen Verfahrens wird das im Schritt a) erhaltene Alkalimetallethanolat mit einem Stabilisator S vermischt.

Dabei bedeutet "das im Schritt a) erhaltene Alkalimetallethanolat" insbesondere, dass der Stabilisator **S** so schnell wie möglich mit dem Alkalimetallethanolat vermischt wird, nachdem dieses im Schritt a) des erfindungsgemäßen Verfahrens erhalten wurde. Insbesondere wird demnach das in Schritt a) des erfindungsgemäßen Verfahrens erhaltene Alkalimetallethanolat direkt nach Beendigung des Schrittes a) mit dem Stabilisator **S** vermischt oder in ein Behältnis (wie zum Beispiel einen Lagertank) gegeben, in dem sich schon der Stabilisator **S** befindet, wo das Alkalimetallethanolat dann mit **S** vermischt wird.

Dies ist eine bevorzugte Ausführungsform der vorliegenden Erfindung, dennoch ist die Erfindung nicht darauf beschränkt, denn der stabilisierende Effekt des Stabilisators S kommt natürlich auch dann zum Tragen, wenn das in Schritt a) erhaltene Alkalimetallethanolat erst ohne Stabilisator stehen gelassen (also gelagert) wird und erst nach Verstreichen eines Zeitraums mit dem Stabilisator **S** vermischt wird. Dann hat zwar schon eine ungehinderte Vergilbung eingesetzt, aber zumindest ab Zugabe des Stabilisators **S** wird die weitere Vergilbung dann verlangsamt oder gar rückgängig gemacht.

Dabei ist der Stabilisator **S** ausgewählt aus einer der folgenden Gruppen I bis VII:
1) Gruppe I: Verbindungen **(I-A)** der Struktur NH₂R¹ und Salze **(I-B)** umfassend das Kation [H₃NR¹]^{m+},
   wobei m eine ganze Zahl ≥ 1 ist,
   und wobei R¹ ausgewählt aus der Gruppe bestehend aus -NH-Phenyl, -Phenyl, -(C=O)-NH₂, -OH, verzweigter oder unverzweigter Alkylrest, der insbesondere 1 bis 10 Kohlenstoffatome aufweisen kann, in welchem mindestens ein Wasserstoffatom durch eine Gruppe ausgewählt aus -NH₂, -OH und im Falle der Salze **(I-B)** auch durch -NH₃⁺ substituiert sein kann, ist.
   m gibt die Anzahl der positiven Ladungen des Kations [H₃NR¹]^{m+} in den Salzen **(I-B)** an. Sie ist m = 1, wenn der Rest R¹ keine NH₃⁺-Gruppe aufweist, und ändert sich, wenn R¹ ein Alkylrest ist, in dem ein Wasserstoffatom durch eine -NH₃⁺-Gruppe substituiert ist. Dementsprechend erhöht sich dann m für jede solche vorhandene -NH₃⁺-Gruppe um den Wert 1.
   In einer bevorzugten Ausführungsform der vorliegenden Erfindung umfasst die Gruppe I Verbindungen **(I-A)** der Struktur NH₂R¹ und Salze **(I-B)** umfassend das Kation [H₃NR¹]^{m+} mit m = 1, wobei R¹ ausgewählt aus der Gruppe bestehend aus -NH-Phenyl, -Phenyl, -(C=O)-NH₂, -OH, verzweigter oder unverzweigter Alkylrest mit 1 bis 10 Kohlenstoffatomen, in welchem mindestens ein Wasserstoffatom durch eine Gruppe ausgewählt aus -NH₂, -OH substituiert sein kann, ist.
   In einer bevorzugteren Ausführungsform der vorliegenden Erfindung umfasst die Gruppe I Verbindungen **(I-A)** der Struktur NH₂R¹ und Salze **(I-B)** umfassend das Kation [H₃NR¹]^{m+} mit m = 1, wobei R¹ ausgewählt aus der Gruppe bestehend aus -NH-Phenyl, -Phenyl, -(C=O)-NH₂, -OH, verzweigter oder unverzweigter, noch bevorzugter unverzweigter, Alkylrest mit 1 bis 6 Kohlenstoffatomen, in welchem mindestens ein Wasserstoffatom durch eine Gruppe ausgewählt aus -NH₂, -OH substituiert sein kann, ist.
   In einer besonders bevorzugten Ausführungsform der vorliegenden Erfindung umfasst die Gruppe I Verbindungen **(I-A)** ausgewählt aus der Gruppe bestehend aus Phenylhydrazin (CAS-Nr.: 100-63-0), Anilin (CAS-Nr.: 62-53-3), Harnstoff (CAS-Nr.: 57-13-6), Alkanolamine mit 1 bis 6 Kohlenstoffatomen, Diaminen mit 1 bis 6 Kohlenstoffatomen und das Salz **(I-B),** welches ein Hydroxylammoniumsalz, besonders bevorzugt Hydroxylammoniumsulfat (CAS-Nr.: 10039-54-0), ist.
   In einer ganz besonders bevorzugten Ausführungsform der vorliegenden Erfindung umfasst die Gruppe I Verbindungen **(I-A)** ausgewählt aus der Gruppe bestehend aus Alkanolamine mit 1 bis 6 Kohlenstoffatomen, Diaminen mit 1 bis 6 Kohlenstoffatomen und das Salz **(I-B),** welches ein Hydroxylammoniumsalz, besonders bevorzugt Hydroxylammoniumsulfat (CAS-Nr.: 10039-54-0), ist.
   Alkanolamine mit 1 bis 6 Kohlenstoffatomen haben bevorzugt 1 bis 4, noch bevorzugter 1 bis 3 Kohlenstoffatome, und noch bevorzugter handelt es sich dabei um Ethanolamin (CAS-Nr.: 141-43-5).
   Diamine mit 1 bis 6 Kohlenstoffatomen haben bevorzugt 1 bis 4, noch bevorzugter 1 bis 3 Kohlenstoffatome, und noch bevorzugter handelt es sich dabei um Ethylendiamin (CAS-Nr.: 107-15-3).
2) Gruppe II: Verbindungen **(II-A)** der Struktur NR²R³R⁹ und Salze **(II-B)** umfassend das Kation [HNR²R³R⁹]ⁿ⁺,
   wobei n eine ganze Zahl ≥ 1 ist,
   und wobei R², R³ unabhängig voneinander jeweils ein Alkylrest, bevorzugt mit 1 bis 10 Kohlenstoffatomen, sind,
   und wobei R⁹ ausgewählt aus der Gruppe bestehend aus -OH, verzweigter oder unverzweigter Alkylrest, bevorzugt mit 1 bis 10 Kohlenstoffatomen, in welchem mindestens ein Wasserstoffatom durch eine Gruppe ausgewählt aus -NH₂, -OH und im Falle der Salze **(II-B)** auch durch -NH₃⁺ substituiert sein kann, ist.
   n gibt die Anzahl der positiven Ladungen des Kations [HNR²R³R⁹]ⁿ⁺, in den Salzen **(II-B)** an. Sie ist n = 1, wenn der Rest R⁹ keine NH₃⁺-Gruppe aufweist, und ändert sich, wenn R⁹ ein verzweigter oder unverzweigter Alkylrest ist, in dem ein Wasserstoffatom durch eine -NH₃⁺-Gruppe substituiert ist. Dementsprechend erhöht sich dann n für jede solche vorhandene -NH₃⁺-Gruppe um den Wert 1.
   In einer bevorzugten Ausführungsform der vorliegenden Erfindung umfasst die Gruppe II Verbindungen **(II-A)** der Struktur NR²R³R⁹ und Salze **(II-B)** umfassend das Kation [HNR²R³R⁹]ⁿ⁺ mit n = 1, wobei R², R³ unabhängig voneinander jeweils ein Alkylrest mit 1 bis 6, bevorzugter mit 1 bis 4, noch bevorzugter mit 1 bis 3 Kohlenstoffatomen ist, und wobei R⁹ ausgewählt aus der Gruppe bestehend aus -OH, verzweigter oder unverzweigter Alkylrest, bevorzugt mit 1 bis 6 Kohlenstoffatomen, in welchem genau ein Wasserstoffatom durch eine Gruppe ausgewählt aus -NH₂, -OH substituiert sein kann, ist.
   In einer bevorzugteren Ausführungsform der vorliegenden Erfindung umfasst die Gruppe II Verbindungen **(II-A)** der Struktur NR²R³R⁹ und Salze **(II-B)** umfassend das Kation [HNR²R³R⁹]ⁿ⁺ mit n = 1, wobei R², R³ unabhängig voneinander jeweils ein Alkylrest mit 1 bis 4, bevorzugter mit 1 bis 3, noch bevorzugter mit 2 Kohlenstoffatomen ist, und wobei R⁹ ausgewählt aus der Gruppe bestehend aus -OH, verzweigter oder unverzweigter Alkylrest, bevorzugt mit 2 bis 3 Kohlenstoffatomen, in welchem genau ein Wasserstoffatom durch eine Gruppe ausgewählt aus -NH₂, -OH, bevorzugt mit -NH₂, substituiert sein kann, ist.
   In einer noch bevorzugteren Ausführungsform der vorliegenden Erfindung umfasst die Gruppe II Verbindungen **(II-A)** der Struktur NR²R³R⁹ und Salze **(II-B)** umfassend das Kation [HNR²R³R⁹]ⁿ⁺ mit n = 1, wobei R², R³ unabhängig voneinander jeweils ein Alkylrest mit 1 bis 4, bevorzugter mit 1 bis 3, noch bevorzugter mit 2 Kohlenstoffatomen ist, und R⁹ ausgewählt aus der Gruppe bestehend aus -OH, Ethyl, 1- Hydroxyethyl, 2-Hydroxyethyl, Propyl, 1-Hyroxypropyl, 2-Hydroxypropyl, 3-Hydroxypropyl, 1-Aminoethyl, 2-Aminoethyl, 1-Aminopropyl, 2-Aminopropyl, 3-Aminopropyl ist.
   In einer noch bevorzugteren Ausführungsform der vorliegenden Erfindung sind Verbindungen der Gruppe II die Verbindungen **(II-A)** der Struktur NR²R³R⁹ ausgewählt aus N,N-Diethylhydroxylamin (CAS-Nr.: 3710-84-7), Diethylaminopropylamin (CAS-Nr.: 104-78-9), noch bevorzugter Diethylaminopropylamin.
3) Gruppe III: Verbindungen **(III-A)** der Struktur R⁴-COOR⁵, Carboxylate **(III-B)** der Struktur R⁴-COO⁻, sowie Orthoester **(III-C)** der Struktur R⁴-C(OR⁶)₃,
   wobei R⁴ ausgewählt aus der Gruppe bestehend aus Wasserstoff, verzweigter oder unverzweigter Alkyl- oder Alkenylrest, insbesondere mit 1 bis 10 Kohlenstoffatomen, in welchem mindestens ein an ein Kohlenstoffatom gebundenes Wasserstoffatom durch einen Rest ausgewählt aus der Gruppe bestehend aus -SH, -SCH₃, -COOR*, -NH₂ und im Falle der Carboxylate **(III-B)** auch durch -NH₃⁺, -COO⁻ substituiert sein kann, ist,
   und wobei R⁵, R* unabhängig voneinander jeweils Wasserstoff oder ein Alkylrest, insbesondere mit 1 bis 10 Kohlenstoffatomen, sind,
   und wobei R⁶ ein Alkylrest, insbesondere mit 1 bis 10 Kohlenstoffatomen, ist.
   In einer bevorzugten Ausführungsform der vorliegenden Erfindung umfasst die Gruppe III Verbindungen **(III-A)** der Struktur R⁴-COOR⁵, Carboxylate **(III-B)** der Struktur R⁴-COO⁻, sowie Orthoester **(III-C)** der Struktur R⁴-C(OR⁶)₃, wobei R⁴ ausgewählt aus der Gruppe bestehend aus Wasserstoff, verzweigter oder unverzweigter Alkyl- oder Alkenylrest, mit 1 bis 6, noch bevorzugter 1 bis 5, noch bevorzugter 1 bis 4 Kohlenstoffatomen, in welchem mindestens ein an ein Kohlenstoffatom gebundenes Wasserstoffatom durch einen Rest ausgewählt aus der Gruppe bestehend aus -SH, -COOR*, -NH₂ und im Falle der Carboxylate **(III-B)** auch durch -NH₃⁺, -COO⁻ substituiert sein kann, ist,
   und wobei R⁵, R* unabhängig voneinander jeweils Wasserstoff oder ein Alkylrest mit 1 bis 6, bevorzugt 1 bis 4, Kohlenstoffatomen, ist,
   und wobei R⁶ ein Alkylrest mit 1 bis 6, insbesondere 1 bis 4 Kohlenstoffatomen, ist.
   In einer noch bevorzugteren Ausführungsform der vorliegenden Erfindung umfasst die Gruppe III Verbindungen **(III-A)** der Struktur R⁴-COOR⁵, Carboxylate **(III-B)** der Struktur R⁴-COO⁻, sowie Orthoester **(III-C)** der Struktur R⁴-C(OR⁶)₃, wobei R⁴ ausgewählt aus der Gruppe bestehend aus Wasserstoff, verzweigter oder unverzweigter Alkyl- oder Alkenylrest, mit 1 bis 3, bevorzugter 1 bis 2 Kohlenstoffatomen, in welchem mindestens ein an ein Kohlenstoffatom gebundenes Wasserstoffatom durch einen Rest ausgewählt aus der Gruppe bestehend aus -SH, -COOR*, -NH₂ und im Falle der Carboxylate **(III-B)** auch durch -NH₃⁺, -COO⁻ substituiert sein kann, ist,
   und wobei R⁵, R* unabhängig voneinander jeweils Wasserstoff, Methylrest oder Ethylrest, bevorzugt Wasserstoff oder Methylrest, sind,
   und wobei R⁶ ein Methylrest ist.
   In einer noch bevorzugteren Ausführungsform der vorliegenden Erfindung sind Verbindungen der Gruppe III ausgewählt aus Trimethylorthoformiat (CAS-Nr.:149-73-5), Glycin (CAS-Nr.: 56-40-6), Mercaptoessigsäure (CAS-Nr.: 68-11-1), Malonsäuredimethylester (CAS-Nummer: 108-59-8), Maleinsäure (CAS-Nr.: 110-16-7), Malonsäuremonomethylester sowie das entsprechende Monocarboxylat, z.B. dessen Kaliumsalz (CAS-Nr.: 38330-80-2), Mercaptoacetat, zum Beispiel dessen Ammoniumsalz (CAS-Nr.: 5421-46-5) sowie dessen Natriumsalz (CAS-Nr.: 367-51-1) oder Kaliumsalz (CAS-Nr.: 34452-51-2), wobei das Ammoniumsalz bevorzugt ist, Mercaptobernsteinsäure (CAS-Nr.: 70-49-5). Noch bevorzugter sind Verbindungen der Gruppe III ausgewählt aus Glycin, Mercaptoessigsäure, Malonsäuredimethylester. Ganz besonders bevorzugt ist die Verbindung der Gruppe III Malonsäuredimethylester.
4) Gruppe IV: Salze von Alkalimetallkationen mit dem Anion [HSO₃]⁻ oder mit Anionen der allgemeinen Formeln **(IV-A)** und **(IV-B)** mit 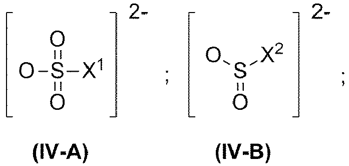 wobei X¹ und X² unabhängig voneinander jeweils ausgewählt sind aus der Gruppe bestehend aus -O, -S, ; 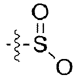 sind, und wobei X¹ auch die Bedeutung 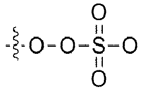 haben kann;
   In einer bevorzugten Ausführungsform der vorliegenden Erfindung umfasst die Gruppe IV Salze von Alkalimetallkationen ausgewählt aus Kaliumionen oder Natriumionen, bevorzugt Natriumionen, mit dem Anion [HSO₃]⁻ oder mit Anionen der allgemeinen Formeln **(IV-A)** und **(IV-B)** mit 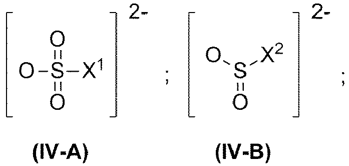 wobei X¹ und X² unabhängig voneinander jeweils ausgewählt sind aus der Gruppe bestehend aus -O, -S, ; 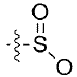 sind, und wobei X¹ auch die Bedeutung 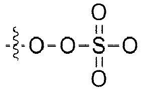 haben kann.
   In einer noch bevorzugteren Ausführungsform sind die Verbindungen der Gruppe IV ausgewählt aus Natriumsulfit (CAS-Nr: 7757-83-7), Natriummetabisulfit (CAS-Nr.: 7681-57-4), Natriumdithionit (CAS-Nr.: 7775-14-6), Natriumthiosulfat (bevorzugt jenes Natriumthiosulfat mit CAS-Nr.: 7772-98-7; aber auch das entsprechende Pentahydrat mit CAS-Nr.: 10102-17-7 ist möglich), Natriumperoxodisulfat (CAS-Nr.: 7775-27-1), Natriumbisulfit (CAS-Nr.: 7631-90-5). Noch bevorzugter sind die Verbindungen der Gruppe IV ausgewählt aus Natriumsulfit und Natriumthiosulfat. Ganz besonders bevorzugt ist die Verbindung der Gruppe IV Natriumthiosulfat.
5) Gruppe V: Alkylthiole, in denen ein an ein Kohlenstoffatom gebundenes Wasserstoffatom durch eine gegebenenfalls alkylierte und gegebenenfalls protonierte Aminogruppe ersetzt sein kann. Bevorzugt handelt es sich dabei um Alkylthiole mit 1 bis 18, noch bevorzugter 2 bis 12 Kohlenstoffatomen in denen ein an ein Kohlenstoffatom gebundenes Wasserstoffatom durch eine gegebenenfalls alkylierte und gegebenenfalls protonierte Aminogruppe ersetzt sein kann. Noch bevorzugter handelt es sich dabei um Alkylthiole mit 1 bis 18, noch bevorzugter 2 bis 12 Kohlenstoffatomen in denen ein an ein Kohlenstoffatom gebundenes Wasserstoffatom durch -NH₂ oder -NH₃⁺ ersetzt sein kann.
   Besonders bevorzugt handelt es sich dabei um Dodecanthiol (CAS-Nr.: 112-55-0) oder Cysteamin (CAS-Nr.: 60-23-1; es kann auch dessen Hydrochlorid mit CAS-Nr. 156-57-0 eingesetzt werden), besonders bevorzugt um Dodecanthiol.
6) Gruppe VI: Formaldehyd, insbesondere eingesetzt als wässrige Formaldehydlösung, Formalin (CAS-Nr.: 82115-62-6) oder Paraformaldehyd (CAS-Nr.: 30525-89-4), ganz besonders bevorzugt Paraformaldehyd (CAS-Nr.: 30525-89-4).
7) Gruppe VII : Zucker, welche bevorzugt aus Aldohexosen, Ketohexosen und Disacchariden ausgewählt sind, wobei der Zucker bevorzugt ausgewählt aus der Gruppe bestehend aus Saccharose (CAS-Nr.: 57-50-1), D-Glucose (CAS-Nr.: 50-99-7), D-Fructose (CAS-Nr.: 57-48-7), L-Glucose (CAS-Nr.: 921-60-8), L-Fructose (CAS-Nr.: 7776-48-9) ist, besonders bevorzugt aber D-Glucose ist.

Besonders bevorzugt ist der Stabilisator **S** ausgewählt aus den Gruppen I, II und III, bevorzugter aus den Gruppen I und II, noch bevorzugter aus der Gruppe I.

Der Stabilisator **S** wird in Schritt b) des erfindungsgemäßen Verfahrens insbesondere in einer solchen Menge zugegeben, dass das Gesamtgewicht aller Stabilisatoren **S** bezogen auf das Gesamtgewicht des Alkalimetallethanolats im Bereich 1 ppm bis 10000 ppm, bevorzugt 10 ppm bis 1000 ppm, noch bevorzugter 100 ppm bis 1000 ppm, am bevorzugtesten 500 ppm bis 800 ppm, liegt.

In einem weiteren Aspekt umfasst die vorliegende Erfindung auch ein Verfahren zur Lagerung von Alkalimetallethanolat, dadurch gekennzeichnet, dass man Alkalimetallethanolat mit einem Stabilisator **S,** wie er in den Gruppen I bis VII definiert ist, mischt, so dass man eine Mischung **M** umfassend Alkalimetallethanolat und den Stabilisator **S** erhält, und die resultierende Mischung **M** lagert.

Das Alkalimetallethanolat im Verfahren zur Lagerung von Alkalimetallethanolat ist bevorzugt ausgewählt aus Natriumethanolat, Kaliumethanolat besonders bevorzugt aber Natriumethanolat.

"Lagerung" bedeutet insbesondere, dass die resultierende Mischung **M** bei einer Temperatur und einem Druck (zum Beispiel 25 °C und 1 bar), bei dem die Mischung **M** flüssig ist, aufbewahrt wird, zum Beispiel in einem Lagertank. Auch hier kommt der stabilisierende Effekt des Stabilisators **S** dadurch zum Tragen, dass die Vergilbung der Mischung **M** verlangsamt oder sogar rückgängig gemacht wird. Insbesondere liegt in der Mischung **M** das Gesamtgewicht aller Stabilisatoren **S** bezogen auf das Gesamtgewicht des Alkalimetallethanolats im Bereich 1 ppm bis 10000 ppm, noch bevorzugter 100 ppm bis 1000 ppm, am bevorzugtesten 500 ppm bis 800 ppm. Die folgenden Beispiele sollen die Erfindung weiter erläutern, ohne diese darauf zu beschränken.

### Beispiele

Das in den Stabilisatorversuchen verwendete Natriumethanolat wurde frisch per Amalgamverfahren gewonnen und wies eine Konzentration von 21 Gew.-% in Ethanol (im Folgenden mit "NE 21" abgekürzt) auf. Es wies eine Gardnerzahl von 1,1 auf, bestimmt mit DIN 4630.

Zur Durchführung der Stabilisatorversuche wurden gleiche Volumina der NE 21 Lösung in GC-Vials gefüllt und jeweils 0.1 Gew.-% des jeweiligen Additivs (bezogen auf die theoretische Masse des NE 21 in der Probe) zugegeben. Daneben wurde auch eine Blindprobe vorbereitet. Die Proben wurden unter N₂ bei Raumtemperatur für 7 Tage bzw. 14 Tagen gelagert.
Nach diesem Zeitraum wurde die Gardnerzahl der jeweiligen Probe gemäß DIN 4630 vermessen, welche ein Maß für die Verfärbung der jeweiligen Probe gibt.

Es wurden 2 Ansatzreihen (Analyse Gardnerzahl nach 7 und 14 Tagen) angesetzt. Zur Messung der Gardnerzahl wurde die zur Messung notwendige Menge NE 21 in eine 1 cm Messküvette gefüllt. Die Messung selbst erfolgte mit einem Photometer der Fa. Lange; Typ: "Lico 400 spektrales Farbmessgerät".
Die folgende Tabelle stellt die für die verschiedenen Stoffklassen gemessenen Werte dar, geordnet gemäß den oben angegebenen Gruppen I bis VII. In den Klammern hinter Substanz ist die CAS-Nummer der jeweiligen Substanz angegeben. **V1** ist das Vergleichsbeispiel, E1 bis E26 sind die erfindungsgemäßen Beispiele.

| **Beispiel** | **Additiv** | Gardnerzahl nach 7 Tagen | Gardnerzahl nach 14 Tagen |
|---|---|---|---|
| **V1** | keines (Blindprobe) | 4,6 | 4,9 |

| **Gruppe I** | | | |
|---|---|---|---|
| **E1** | Anilin (62-53-3) | 1,8 | 1,8 |
| **E2** | Phenylhydrazin (100-63-0) | 2,7 | 1,8 |
| **E3** | Harnstoff (57-13-6) | 4,0 | 4,1 |
| **E4** | Ethanolamin (141-43-5) | 0,7 | 0,7 |
| **E5** | Ethylendiamin (107-15-3) | 0,6 | 0,6 |
| **E6** | Hydroxylammoniumsulfat (10039-54-0) | 0,6 | 0,5 |

| **Gruppe II** | | | |
|---|---|---|---|
| **E7** | *N,N*-Diethylhydroxylamin (3710-84-7) | 2,9 | 3,3 |
| **E8** | Diethylaminopropylamin (104-78-9) | 0,9 | 1,0 |

| **Gruppe III** | | | |
|---|---|---|---|
| **E9** | Trimethylorthoformiat (149-73-5) | 4,1 | 3,8 |
| **E10** | Mercaptoessigsäure (68-11-1) | 2,6 | 2,7 |
| **E11** | Malonsäuredimethylester (108-59-8) | 1,0 | 1,0 |
| **E12** | Glycin (56-40-6) | 3,6 | 2,4 |
| **E13** | Maleinsäure (110-16-7) | 4,2 | 3,9 |
| **E14** | Malonsäuremonomethylester, Kaliumsalz (38330-80-2) | 4,5 | 4,8 |
| **E15** | Ammoniumthioglycolat (5421-46-5) | 2,5 | 3,6 |
| **E16** | Mercaptobernsteinsäure (70-49-5) | 4,0 | 4,2 |

| **Gruppe IV** | | | |
|---|---|---|---|
| **E17** | Natriumsulfit (7757-83-7) | 4,3 | 3,9 |
| **E18** | Natriummetabisulfit (7681-57-4) | 4,3 | 4,3 |
| **E19** | Natriumdithionit (7775-14-6) | 3,8 | 4,3 |
| **E20** | Natriumthiosulfat (7772-98-7) | 4,2 | 3,7 |
| **E21** | Natriumperoxodisulfat (7775-27-1) | 4,3 | 4,5 |
| **E22** | Natriumbisulfit (7631-90-5) | 4,5 | 4,5 |

| **Gruppe V** | | | |
|---|---|---|---|
| **E23** | Cysteaminhydrochlorid (156-57-0) | 3,2 | 3,1 |
| **E24** | Dodecanthiol (112-55-0) | 2,7 | 2,7 |

| **Gruppe VI** | | | |
|---|---|---|---|
| **E25** | Paraformaldehyd (30525-89-4) | 2,8 | 2,3 |

| **Gruppe VII** | | | |
|---|---|---|---|
| **E26** | D-Glucose (50-99-7) | 3,7 | 4,2 |

Wie aus der Tabelle ersichtlich, zeichnen sich alle Stabilisatoren dadurch aus, dass diese bei Zugabe zu Natriumethanolat die Verfärbung desselben stark verlangsamen und in einigen Fällen (Verminderung der Gardnerzahl von 1,1 auf < 1,1 in den Versuchen **E4** - **E6, E8, E11**) sogar rückgängig machen. Dieses Ergebnis war völlig überraschend.

## Patentansprüche

1. Verfahren zur Herstellung von Alkalimetallethanolat mit hoher Farbstabilität, wobei man
a) Alkalimetall mit Ethanol umsetzt, wodurch Alkalimetallethanolat erhalten wird;
b) das in Schritt a) erhaltene Alkalimetallethanolat mit einem Stabilisator S vermischt, wobei S ausgewählt aus der Gruppe bestehend aus
Verbindungen **(I-A)** der Struktur NH₂R¹ und Salze **(I-B)** umfassend das Kation [H₃NR¹]^{m+},
wobei m eine ganze Zahl ≥ 1 ist,
und wobei R¹ ausgewählt aus der Gruppe bestehend aus -NH-Phenyl, -Phenyl, -(C=O)-NH₂, -OH, verzweigter oder unverzweigter Alkylrest, in welchem mindestens ein Wasserstoffatom durch eine Gruppe ausgewählt aus -NH₂, -OH und im Falle der Salze **(I-B)** auch durch -NH₃⁺ substituiert sein kann, ist,
Verbindungen **(II-A)** der Struktur NR²R³R⁹ und Salze **(II-B)** umfassend das Kation [HNR²R³R⁹]ⁿ⁺,
wobei n eine ganze Zahl ≥ 1 ist,
und wobei R², R³ unabhängig voneinander jeweils ein Alkylrest sind,
und wobei R⁹ ausgewählt aus der Gruppe bestehend aus -OH, verzweigter oder unverzweigter Alkylrest, in welchem mindestens ein Wasserstoffatom durch eine Gruppe ausgewählt aus -NH₂, -OH und im Falle der Salze **(II-B)** auch durch -NH₃⁺ substituiert sein kann, ist,
Verbindungen **(III-A)** der Struktur R⁴-COOR⁵, Carboxylate **(III-B)** der Struktur R⁴-COO⁻, sowie Orthoester **(III-C)** der Struktur R⁴-C(OR⁶)₃,
wobei R⁴ ausgewählt aus der Gruppe bestehend aus Wasserstoff, verzweigter oder unverzweigter Alkyl- oder Alkenylrest, in welchem mindestens ein an ein Kohlenstoffatom gebundenes Wasserstoffatom durch einen Rest ausgewählt aus der Gruppe bestehend aus -SH, -SCH₃, -COOR*, -NH₂ und im Falle der Carboxylate **(III-B)** auch durch -NH₃⁺, -COO⁻ substituiert sein kann, ist,
und wobei R⁵, R* unabhängig voneinander jeweils Wasserstoff oder ein Alkylrest sind, und wobei R⁶ ein Alkylrest ist,
Salze von Alkalimetallkationen mit dem Anion [HSO₃]⁻ oder mit Anionen der allgemeinen Formeln **(IV-A)** und **(IV-B)** mit 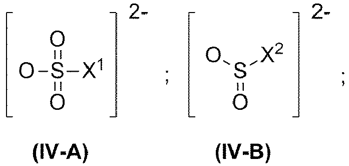 wobei X¹ und X² unabhängig voneinander jeweils ausgewählt sind aus der Gruppe bestehend aus -O, -S, ; 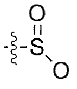 sind, und wobei X¹ auch die Bedeutung 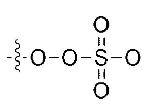 haben kann,
Alkylthiolen, in denen ein an ein Kohlenstoffatom gebundenes Wasserstoffatom durch eine gegebenenfalls alkylierte und gegebenenfalls protonierte Aminogruppe ersetzt sein kann,
Formaldehyd,
Zucker,
ist.

2. Verfahren nach Anspruch 1, wobei der Stabilisator S ausgewählt ist aus Verbindungen **(I-A)** der Struktur NH₂R¹ und Salze **(I-B)** umfassend das Kation [H₃NR¹]^{m+}, Verbindungen **(II-A)** der Struktur NR²R³R⁹ und Salze **(II-B)** umfassend das Kation [HNR²R³R⁹]ⁿ⁺, Verbindungen **(III-A)** der Struktur R⁴-COOR⁵, Carboxylate **(III-B)** der Struktur R⁴-COO⁻, sowie Orthoester **(III-C)** der Struktur R⁴-C(OR⁶)₃.

3. Verfahren nach Anspruch 1 oder 2, wobei das Alkalimetallethanolat als ethanolische Lösung vorliegt.

4. Verfahren nach einem der Ansprüche 1 bis 3, wobei das Alkalimetall ausgewählt aus der Gruppe bestehend aus Natrium, Kalium ist.

5. Verfahren nach einem der Ansprüche 1 bis 4, wobei das Alkalimetall im Schritt a) als Alkalimetallamalgam eingesetzt wird.

6. Verfahren nach Anspruch 5, wobei der Schritt a) in Gegenwart eines heterogenen Katalysators durchgeführt wird.

7. Verfahren nach Anspruch 6, wobei der heterogene Katalysator mindestens Graphit und/oder ein Metall ausgewählt aus der Gruppe Aluminium, Übergangsmetall umfasst.

8. Verfahren nach einem der Ansprüche 1 bis 7, wobei in Schritt b) der Stabilisator **S** in einer solchen Menge zugegeben wird, dass das Gesamtgewicht aller Stabilisatoren **S,** bezogen auf das Gesamtgewicht des Alkalimetallethanolats, im Bereich 1 ppm bis 10000 ppm liegt.

9. Verfahren zur Lagerung von Alkalimetallethanolat, **dadurch gekennzeichnet, dass** man Alkalimetallethanolat mit einem Stabilisator **S,** wie er in Anspruch 1 oder 2 definiert ist, mischt, so dass man eine Mischung **M** umfassend Alkalimetallethanolat und den Stabilisator **S** erhält, und die resultierende Mischung **M** lagert.

10. Verfahren nach Anspruch 9, wobei in der Mischung **M** das Gesamtgewicht aller Stabilisatoren **S** bezogen auf das Gesamtgewicht des Alkalimetallethanolats im Bereich 1 ppm bis 10000 ppm liegt.
